(19) Europäisches Patentamt
European Patent Office
Office européen des brevets

(11) Veröffentlichungsnummer : **0 348 849 B1**

(12) # EUROPÄISCHE PATENTSCHRIFT

(45) Veröffentlichungstag der Patentschrift : **14.10.92 Patentblatt 92/42**

(51) Int. Cl.$^5$ : **A61K 7/13**

(21) Anmeldenummer : **89111507.3**

(22) Anmeldetag : **23.06.89**

(54) **Haarfärbemittel.**

Verbunden mit 89907751.5/0423181 (europäische Anmeldenummer/Veröffentlichungsnummer) durch Entscheidung vom 15.10.91.

(30) Priorität : **01.07.88 DE 3822365**

(43) Veröffentlichungstag der Anmeldung : **03.01.90 Patentblatt 90/01**

(45) Bekanntmachung des Hinweises auf die Patenterteilung : **14.10.92 Patentblatt 92/42**

(84) Benannte Vertragsstaaten : **AT BE CH DE ES FR GB IT LI NL SE**

(56) Entgegenhaltungen :
**EP-A- 0 181 505**
**EP-A- 0 211 238**
**DE-A- 2 447 017**
**PATENT ABSTRACTS OF JAPAN, Band 10, Nr. 350 (C-387)[2406], 26. November 1986**

(73) Patentinhaber : **Henkel Kommanditgesellschaft auf Aktien**
**Postfach 1100 Henkelstrasse 67**
**W-4000 Düsseldorf-Holthausen (DE)**

(72) Erfinder : **Rose, David, Dr.**
**Am Eichelkamp 223**
**W-4010 Hilden (DE)**
Erfinder : **Höffkes, Horst, Dr.**
**Carlo-Schmid-Strasse 113**
**W-4000 Düsseldorf (DE)**
Erfinder : **Lieske, Edgar**
**Hunsrückenstrasse 40**
**W-4000 Düsseldorf (DE)**

**EP 0 348 849 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferner ein gutes Aufziehvermögen auf menschlichem Haar besitzen, ohne die Kopfhaut zu stark anzufärben. Auch soll der Farbaufzug gleichmäßig erfolgen, d.h. die stärker strapazierten Haarspitzen sollen nicht stärker gefärbt werden als der wenig geschädigte Haaransatz. Die damit erzeugten Färbungen müssen eine hohe Stabilität gegen Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

3-Aminophenole sind als Oxidationsfarbstoffvorprodukte z. B. aus DE-AS 11 43 605, DE-AS 11 51 900, DE 24 47 017 C2 und DE 30 16 882 A1 schon bekannt. Die mit diesen Produkten und bekannten Entwicklerkomponenten herstellbaren Haarfärbemittel befriedigen jedoch nicht in bezug auf die Echtheitseigenschaften der damit erzielbaren Haaranfärbungen. Insbesondere erhält man mit den bekannten 3-Aminophenolen keine braunen Nuancen von befriedigender Natürlichkeit, Brillanz und Farbtiefe.

Es wurde gefunden, daß Haarfärbemittel, enthaltend Oxidationsfarbsotffvorprodukte in einem Träger, die als Oxidationsfarbstoffvorprodukte N-Cycloalkyl-3-aminophenole der Formel (I)

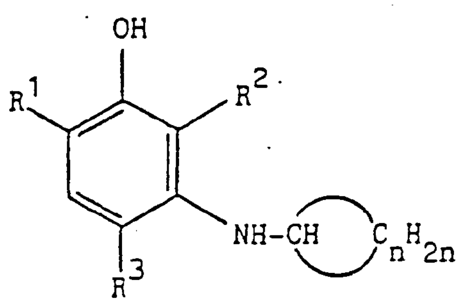

in der n eine ganze Zahl von 4 - 8 und $R^1$, $R^2$ und $R^3$ unabhängig voneinander Wasserstoff, Methylgruppen oder Chlor sind, oder deren Salze als Kupplersubstanzen und die in Oxidationshaarfärbemitteln üblichen Entwicklersubstanzen enthalten, die gestellten Anforderungen in hohem Maße erfüllen.

Bevorzugt, besonders wegen ihrer guten Zugänglichkeit, werden solche N-Cycloalkyl-3-aminophenole der Formel I eingesetzt, worin $R^1$, $R^2$ und $R^3$ Wasserstoff und die Gruppe

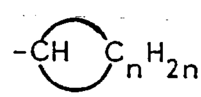

eine gegebenenfalls durch 1 oder 2 Methylgruppen substituierte Cyclopentyl- oder Cyclohexylgruppe ist. Besonders bevorzugt sind die als Beispiele (Kupplerkomponenten K1 K2 und K3) aufgeführten Verbindungen.

Die N-Cycloalkyl-3-aminophenole der Formel I, worin $R^1$, $R^2$ und $R^3$ Wasserstoff bedeuten, sind literaturbekannte Verbindungen. Ihre Herstellung ist z. B. aus EP 181 505 A1 bekannt. Die übrigen N-Cycloalkyl-3-aminophenole der Formel I, worin $R^1$, $R^2$ und $R^3$ Methylgruppen oder Chlor bedeuten, lassen sich auf analoge

Weise nach dem dort angegebenen Verfahren aus dem entsprechenden Aminophenol und einem Cycloalkanon in Gegenwart eines Reduktionsmittels nach folgenden Formelschema herstellen:

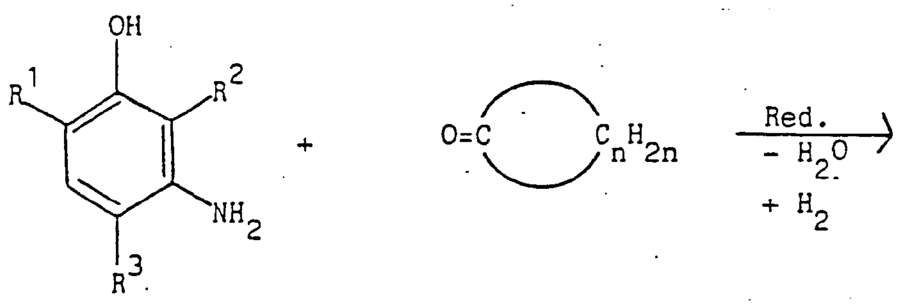


(I)

Geeignete Aminophenole sind neben dem 3-Aminophenol z. B. 5-Chlor-3-aminophenol, 5-Chlor-6-methyl-3-aminophenol, 4-Chlor-3-aminophenol, 5-Methyl-3-aminophenol, 6-Methyl-3-aminophenol, 2-Methyl-6-aminophenol und 2,6-Dimethyl-3-aminophenol. Geeignete Cycloalkanone sind z. B. Cyclopentanon, 3-Methyl-cyclopentanon, Cyclohexanon, 3-Methyl-cyclohexanon, Dimethyl-cyclopentanon, Dimethyl-cyclohexanone, Isopropyl-methyl-cyclohexanon, Trimethylcyclopentanone und Trimethylcyclohexanone oder Cycloheptanon.

Die N-Cycloalkyl-3-aminophenole der Formel I stellen wertvolle Kupplerverbindungen dar, die mit einer Vielzahl üblicher Entwicklerkomponenten intensive Oxidationsfarben von hoher Licht-, Wärme- und Kaltwellechtheit überwiegend im Bereich brauner und blauvioletter Nuancen ausbilden.

Als Entwickler können alle hierfür bekannten Verbindungen eingesetzt werden. Solche Entwicklersubstanzen sind zum Beispiel p-Phenyldiamin, p-Toluylendiamin, N,N-Diethyl-2-methyl-p-phenylendiamin, N-Ethyl-N-(2-hydroxylethyl)-p-phenylendiamin, Chlor-p-phenylendiamin, N,N-Bis-(2-hydroxylethyl)-p-phenylendiamin, N,N-Dimethyl-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, 2,5-Diaminoanisol, N-(2-Hydroxypropyl)-p-phenyldiamin, N-2-Methoxyethyl-p-phenylendiamin, N-Butyl-N-sulfobutyl-p-phenylendiamin und andere Verbindungen der genannten Art, die weiterhin eine oder mehrere $NH_2$-Gruppen, NHR-Gruppen, $NR_2$-Gruppen enthalten können, wobei R einen Alkylrest mit 1 - 4 oder einen Hydroxyalkylrest mit 2 - 4 Kohlenstoffatomen darstellt, ferner p-Aminophenole, Diaminopyridinderivate sowie besonders Tetraaminopyrimidine wie 2,4,5,6-Tetraaminopyrimidin, 4,5-Diamino-2,6-bis-methylaminopyrimidin, 2,5-Diamino-4-diethyl-amino-6-methylamino-pyrimidin, 2,4,5-Triamino-6-piperidino-pyrimidin, 2,4,5-Triamino-6-anilino-pyrimidin, 2,4,5-Triamino-6-morpholinopyrimidin, 2,4,5-Triamino-6-(2-hydroxyethyl)-aminopyrimidin.

Bevorzugt werden als Entwicklersubstanzen p-Phenylendiamin, p-Toluylendiamin oder Derivate dieser Verbindungen oder deren Salze eingesetzt.

Die erfindungsgemäß zu verwendenden N-Cycloalkyl-3-aminophenole der Formel I sowie die als Entwicklerkomponenten geeigneten p-Phenylendiamin- oder p-Toluylendiamin-Derivate können entweder als solche oder in Form ihrer Salze mit anorganischen oder organischen Säuren, zum Beispiel als Chlorid, Sulfat, Phosphat, Acetat, Propionat, Lactat oder Citrat eingesetzt werden.

Die erfindungsgemäßen Haarfärbemittel können neben den N-Cycloalkyl-3-aminophenolen der allgemeinen Formel (I) auch andere bekannte Kupplersubstanzen enthalten, die zur Modifizierung der Farbnuancen und zur Erzeugung natürlicher Farbtöne erforderlich sind. Solche üblichen Kupplerverbindungen sind z. B. andere m-Phenylendiamine, z. B. 2,4-Diaminophenyl-2-hydroxyethylether, oder Phenole, Resorcine, m-Aminophenole, Naphthole oder Pyrazolone. Gegebenenfalls können auch direktziehende Farbstoffe zusätzlich zur weiteren Modifizierung der Farbnuancen eingesetzt werden. Solche direktziehenden Farbstoffe sind z. B. Nitrophenylendiamine, Nitroaminophenole, Anthrachinonfarbstoffe oder Indophenole.

Zu den erfindungsgemäßen Haarfärbemitteln werden die N-Cylcloalkyl-3-aminophenole der Formel (I) und die gegebenenfalls zusätzlich vorhandenen bekannten Kupplersubstanzen im allgemeinen in etwa molaren Mengen, bezogen auf die verwendeten Entwicklersubstanzen, eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklersubstanzen und Kupplersubstanzen in einem Molverhältnis von 1 : 0,5 bis 1 : 2 enthalten sein können.

Es ist nicht erforderlich, daß die N-Cycloalkyl-3-aminophenole der Formel (I) sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler- oder Entwicklersubstanzen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendung auf dem Haar geeignet sind. Übliche Bestandteile solcher kosmetischer Zubereitungen sind z. B. Netz- und Emulgiermittel wie anionische, nichtionische oder ampholytische Tenside, z. B. Seifen, Fettalkoholsulfate, Alkansulfonate, alpha-Olefinsulfonate, Fettalkoholpolyglykolethersulfate, Ethylenoxidanlagerungsprodukte an Fettalkohole, an Fettsäuren und an Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettkomponenten wie z. B. Fettalkohole. Paraffinöle oder Fettsäureester ferner Parfümöle und haarpflegende Zusätze, wie z. B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt , z. B. werden Emulgiermittel in Konzentrationen von 0,5 - 30 Gew.-% und Verdickungsmittel in Konzentrationen von 0, 1 - 25 Gew.-% des gesamten Färbemittels eingesetzt.

Besonders geeignet als Träger ist eine Öl-in-Wasser-Emulsion mit einem Gehalt von 0,1 - 25 Gew.-% einer Fettkomponente und 0,5 - 30 Gew.-% eines Emulgiermittels aus der Gruppe der anionischen, nichtionischen oder ampholytischen Tenside.

Die Oxidationsfarbstoffvorprodukte werden in Mengen von 0,2 - 5 Gew.-%, vorzugsweise 1 - 3 Gew.-% des gesamten Färbemittels in den Träger eingemischt. Der Gehalt an N-Cycloalkyl-3-aminophenolen der Formel (I) kann in den erfindungsgemäßen Haarfärbemitteln etwa 0,05 - 10 Millimol pro 100 g des Haarfärbemittels betragen.

Die Anwendung der erfindungsgemäßen Haarfärbemittel kann, unabhängig von der Art der kosmetischen Zubereitung, z. B. als Creme, Gel oder Shampoo bevorzugt im schwach alkalischen Milieu erfolgen. Bevorzugt ist die Anwendung der Haarfärbemittel in einem pH-Bereich von 6 - 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo verwendet wurde.

Die nachfolgenden Beispiele sollen den Erfindungsgegenstand näher erläutern.

## Beispiele

### Haarfärbeversuche

Es wurden erfindungsgemäße Haarfärbemittel in Form einer Haarfärbe-Cremeemulsion der folgenden Zusammensetzung hergestellt:

| | |
|---|---|
| Fettalkohol $C_{12}$-$C_{18}$ | 10,0 g |
| Fettalkohol $C_{12}$-$C_{14}$ + 2EO-sulfat, Na-Salz, 28 %ig | 25,0 g |
| Wasser | 60,0 g |
| Entwicklerkomponente (Komponente E) | 7,5 mMol |
| Kupplerkomponente (Komponente K) | 7,5 mMol |
| $Na_2SO_3$ (Inhibitor) | 1,0 g |
| konzentrierte Amoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Amoniak-Lösung der pH-Wert der Emulsion

auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Kupplerkomponente (Komponente K) wurden die folgenden N-Cycloalkyl-3-aminophenole eingesetzt:

K1: N-Cyclopentyl-3-aminophenol
K2: N-Cyclohexyl-3-aminophenol
K3: N-(3-Methylcyclohexyl)-3-aminophenol

Als Entwicklerkomponente (Komponente E) wurden die folgenden Verbindungen eingesetzt:

E1: p-Phenylendiamin
E2: p-Toluylendiamin
E3: N,N-Diethyl-p-phenylendiamin
E4: N,N-Bis-(β-hydroxyethyl)-p-phenylendiamin
E5: N-(β-Hydroxyethyl)-p-phenylendiamin
E6: 2-Chlor-p-phenylendiamin
E7: 2,5-Diaminobenzylalkohol
E8: 2-(2,5-Diaminophenyl)ethanol
E9: 1,2-Bis-(4-aminophenyl)-ethylendiamin
E10: 2,5-Diaminophenyl-β-ethoxyethylether
E11: p-Aminophenol
E12: 2,4,5,6-Tetraaminopyrimidin
E13: 2-Dimethylamino-4,5,6-triaminopyrimidin

Die mit diesen Oxidationsfärbemittelvorprodukten in den Kombinationen gemäß Tabelle I enthaltenen Haaranfärbungen sind der Tabelle zu entnehmen.

Tabelle 1

| Haarfärbe-Beispiel Nr. | Entwickler-Komponente | Kuppler-Komponente | erhaltene Farbnuance |
|---|---|---|---|
| 1 | E1 | K1 | aubergine |
| 2 | E2 | K1 | braunschwarz |
| 3 | E3 | K1 | schwarzblau |
| 4 | E4 | K1 | schwarzblau |
| 5 | E5 | K1 | dunkelviolett |
| 6 | E6 | K1 | dunkelbraun |
| 7 | E8 | K1 | graubraun |
| 8 | E11 | K1 | braun |
| 9 | E12 | K1 | blauviolett |
| 10 | E13 | K1 | blaugrau |
| 11 | E2 | K2 | braunschwarz |
| 12 | E12 | K2 | dunkelviolett |
| 13 | E7 | K3 | graubraun |
| 14 | E9 | K3 | graubraun |
| 15 | E10 | K3 | dunkelviolett |

## Patentansprüche

1. Haarfärbemittel, enthaltend Oxidationsfarbstoffvorprodukte in einem Träger, dadurch gekennzeichnet, daß als Oxidationsfarbstoffvorprodukte N-Cycloalkyl-3-aminophenole der Formel (I)

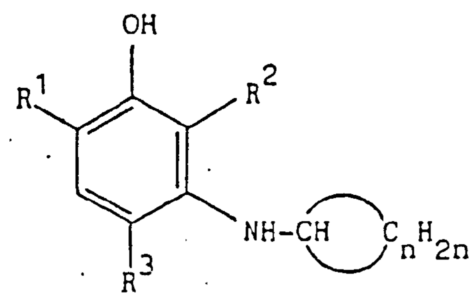

(I)

in der n eine ganze Zahl von 4 - 8 und $R^1$, $R^2$ und $R^3$ unabhängig voneinander Wasserstoff, Methylgruppen oder Chlor sind, oder deren Salze als Kupplersubstanzen und die in Oxidationshaarfärbemitteln üblichen Entwicklersubstanzen enthalten sind.

2. Haarfärbemittel nach Anspruch 1, dadurch gekennzeichnet, daß $R^1$, $R^2$ und $R^3$ Wasserstoff und die Gruppe

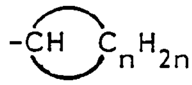

eine gegebenenfalls durch 1 oder 2 Methylgruppen substituierte Cyclopentyl- oder Cyclohexylgruppe ist.

3. Haarfärbemittel nach Anspruch 1 oder 2, dadurch gekennzeichnet, daß als Entwicklersubstanzen p-Phenylendiamin, p-Toluylendiamin oder Derivate dieser Verbindungen oder deren Salze enthalten sind.

4. Haarfärbemittel nach einem der Ansprüche 1 - 3, dadurch gekennzeichnet, daß als Träger eine Öl-in-Wasser-Emulsion mit einem Gehalt von 0,1 - 25 Gew.-% einer Fettkomponente und 0,5 bis 30 Gew.-% eines Emulgiermittels aus der Gruppe der anionischen, nichtionischen oder ampholitischen Tenside und als Oxidationsfarbstoffvorprodukte N-Cycloalkyl-3-aminophenole der Formel I in einer Menge von 0,05 bis 10 Millimol pro 100 g des Haarfärbemittels enthalten sind.

## Claims

1. Hair dyeing preparations containing oxidation dye precursors in a carrier, characterized in that they contain as oxidation dye precursors N-cycloalkyl-3-aminophenols corresponding to formula (I)

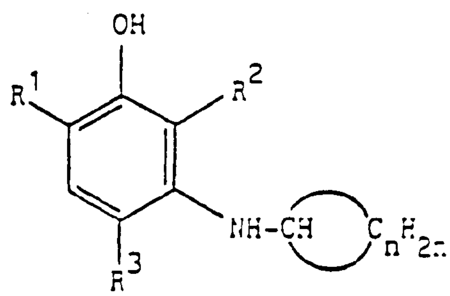

in which n is an integer of 4 to 8 and $R^1$, $R^2$ and $R^3$ independently of one another represent hydrogen, methyl groups or chlorine,
or salts thereof as coupler components and the developer components typically used in oxidation hair dyes.

2. Hair dyeing preparations as claimed in claim 1, characterized in that $R^1$, $R^2$ and $R^3$ represent hydrogen and the group

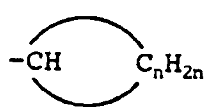

is a cyclopentyl or cyclohexyl group optionally substituted by 1 or 2 methyl groups.

3. Hair dyeing preparations as claimed in claim 1 or 2, characterized in that they contain p-phenylenediamine, p-tolylenediamine or derivatives of these compounds or salts thereof as developer components.

4. Hair dyeing preparations as claimed in any of claims 1 to 3, characterized in that they contain an oil-in-water emulsion containing 0.1 to 25% by weight of a fatty component and 0.5 to 30% by weight of an emulsifier from the group of anionic, nonionic or ampholytic surfactants as carrier and the N-cycloalkyl-3-aminophenols corresponding to formula I in a quantity of 0.05 to 10 millimol per 100 g of the hair dyeing preparation as oxidation dye precursors.

## Revendications

**1.** Teintures pour cheveux contenant des précurseurs de colorants d'oxydation dans un support, caractérisées en ce qu'elles renferment comme précurseurs de colorants d'oxydation, des N-cycloalkyl-3-aminophénols de formule (I)

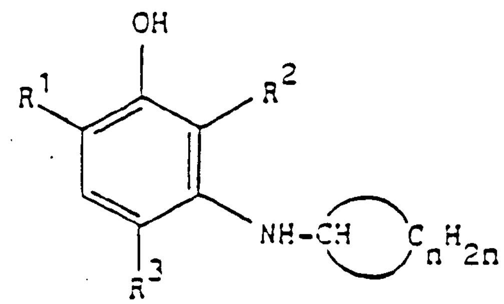

(I)

dans laquelle n représente un nombre entier de 4 à 8 et où $R^1$, $R^2$ et $R^3$ sont indépendamment l'un de l'autre l'hydrogène, des groupes méthyle ou le chlore, ou leurs sels, à titre de copulants ainsi que les substances révélatrices usuelles dans les teintures pour cheveux par oxydation.

**2.** Teintures pour cheveux selon la revendication 1, caractérisées en ce que $R^1$, $R^2$ et $R^3$ représentent l'hydrogène et le groupe

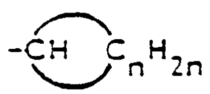

est un groupe cyclopentyle ou cyclohexyle substitué le cas échéant par 1 ou 2 groupes méthyle.

**3.** Teintures pour cheveux selon la revendication 1 ou 2, caractérisées en ce qu'elles renferment, comme substances révélatrices, la p-phénylènediamine, la p-toluylènediamine ou des dérivés de ces composés ou leurs sels.

**4.** Teintures pour cheveux selon l'une des revendications 1 à 3, caractérisées en ce qu'elles renferment comme support, une émulsion huile dans l'eau contenant 0,1 à 25 % en poids d'un composant gras et 0,5 à 30 % en poids d'un émulsifiant appartenant au groupe des tensioactifs anioniques, non ioniques ou ampholytes et, comme précurseurs de colorants d'oxydation, des N-cycloalkyl-3-aminophénols de la formule I, dans une proportion comprise dans l'intervalle de 0,05 à 10 millimoles par 100 g de la teinture pour cheveux.